# EUROPEAN PATENT APPLICATION

(11) **EP 0 768 081 A1**
(43) Date of publication of application: **16.04.1997**
(21) Application number: 96114953.1
(22) Date of filing: 18.09.1996
(51) Int. Cl.: A61K 7/42, A61K 7/50

(54) **Use of an aqueous emulsion or dispersion as hair conditioning composition**

(30) Priority: 13.10.1995 DE 19538094
(71) Applicant: KAO CORPORATION, Chuo-ku, Tokyo (JP)
(72) Inventor: Onitsuka, Satoshi, Oyamashi, Tochigi-ken (JP)
(74) Representative: Hansen, Bernd, Dr. Dipl.-Chem.

(57) **Abstract**

Use of an aqueous emulsion or dispersion as hair conditioning composition, comprising
a) at least one surface-active quaternary ammonium compound;
b) at least one fatty or oily substance; and
c) at least one water-soluble UV absorbent material,
protecting the hair against damage by UV-radiation.

## Description

The invention comprises a new hair conditioning composition, especially for application as a final treatment on shampooed, permanently waved, dyed or styled hair.

Compositions of this kind which, after application either remain on the hair or are rinsed out with water, have been known in the art for a long time; they are intended to provide hold, volume, improved combability and a generally good appearance of the hair.

These compositions, which are usually provided in the form of aqueous emulsions or dispersions, may comprise various different basic and active ingredients. These are especially cationic substances such as surface-active quaternary ammonium compounds, hydrophobic substances such as fatty alcohols or oil components, co-emulsifiers, thickeners such as cellulose derivatives, conditioning actives such as synthetic and natural polymers, humectants, specific active agents, etc.

A survey of such compositions and their use in hair rinsing preparations is found in the monography of K.Schrader, "Grundlagen und Rezepturen der Kosmetika", 2nd Ed. (1989), Hüthig Buchverlag, Heidelberg, pp. 722 to 737.

Moreover, it is well-known to add a sunscreening filter to such compositions to prevent hair damage caused by UV radiation.

European Patent No.419 164 describes a hair conditioning composition which is provided as an aqueous emulsion containing a cation-active surfactant comprising an oily, i.e., water-insoluble UV absorbent material in the lamellar phase of the emulsion.

These compositions, however, still need improvement.

It has now been found that human hair may be provided with excellent protection against damage by sun radiation, and also with enhanced lustre and conditioning effect, by treating with a composition comprising at least one surface-active quaternary ammonium compound, at least one fatty or oily compound, and at least one water-soluble UV absorbent material in an aqueous emulsion or dispersion.

Presumably the use of the water-soluble UV absorbent material in these compositions induces improved substantivity thereof on the hair and consequently increased sun protection.

The conditioning compositions according to the invention contain at least one surface-active quaternary ammonium compound.

Of course, these are well-known per se; suitable long-chain quaternary ammonium compounds which may be used alone or in admixture are particularly cetyl trimethyl ammonium chloride, dimethyl dicetyl ammonium chloride, trimethyl cetyl ammonium bromide, stearyl trimethyl ammonium chloride, dimethyl stearyl benzyl ammonium chloride, benzyl tetradecyl dimethyl ammonium chloride, dimethyl dihydrated tallow ammonium chloride, lauryl pyridinium chloride, lauryl dimethyl benzyl ammonium chloride, lauryl trimethyl ammonium chloride, tris(oligooxyethyl)alkyl ammonium phosphate, cetyl pyridinium chloride, etc.

Well-suitable are also quaternary ammonium salts as disclosed in European Patent Application No. 472,107.

On principle, all quaternary ammonium compounds listed in "CTFA Cosmetic Ingredient Dictionary", Fourth Ed. (1991) under the generic name "Quaternium" may be used.

Particularly suitable according to the invention is a mixture of dicetyl dimethyl ammonium chloride and stearyl trimethyl ammonium chloride, preferably in a weight ratio from 1 : 5 to 5 : 1.

The proportion of these quaternary ammonium compounds in the hair conditioning compositions according to the invention is 0.1% to 10%, preferably 0.25% to 5%, particularly 0.5% to 2.5% by wt., calculated to the total composition.

Suitable fatty and oily substances which also include waxes are in particular natural oils such as avocado oil, coconut oil, palm oil, sesame oil, peanut oil, whale oil, sunflower seed oil, almond oil, peach kernel oil, wheat germ oil, macadamia nut oil, primrose oil, jojoba oil, castor oil, or also olive oil or soybean oil, lanolin and its derivatives, also mineral oils such as paraffin oil and vaseline.

Synthetic oils and waxes, e.g., are silicone oils, polyethylene-glycols, etc.

Other suitable hydrophobic compounds are particularly fatty alcohols, preferably those with about 8 to 22 carbon atoms in the molecule such as myristyl, cetyl, stearyl alcohol, wax alcohols and fatty acid esters such as isopropyl myristate, palmitate, stearate and isostearate, oleyl oleate, isocetyl stearate, hexyl laurate, dibutyl adipate, dioctyl adipate, myristyl myristate, oleyl erucate, polyethyleneglycol and polyglyceryl fatty acid esters such as PEG-7-glyceryl cocoate, cetyl palmitate, etc.

These hydrophobic compounds are incorporated in the compositions according to the invention in a proportion of preferably about 0.5% to about 10%, particularly about 1% to 7.5%, optimally about 1.5% to 5% by wt., calculated to the total composition.

The third essential ingredient of the compositions according to the invention is a water-soluble UV absorbent material used in a proportion of preferably 0.05% to 5%, particularly 0.1% to 2.5%, optimally 0.25% to 1.0% by wt., calculated to the total composition.

Particularly appropriate water-soluble UV absorbent materials are, e.g., 5-benzoyl-4-hydroxy-2-methoxybenzene sulfonic acid (benzophenone-4), its sodium salt (benzophenone-5) and 2,2'-dihydroxy-4,4'-dimethoxy-3,3'-disulfobenzophenone or the disodium salt thereof (benzophenone-9) as well as phenyl benzimidazole sulfonic acid (Eusolex® 232); however, other water-soluble UV absorbent materials may also be used.

The compositions according to the invention may contain further ingredients usual in conditioning treatments.

To avoid repetition, reference is again made to K.Schrader, "Grundlagen und Rezepturen der Kosmetika", 2nd Ed. (1989), l.c., pp. 722 to 771.

Nonionic surfactants may also be used in admixture with cationic surfactants, in a proportion of about 0.5% to about 5%, preferably from about 0.5% to about 3.5% by wt., calculated to the total composition.

Suitable nonionic surfactants, e.g., are amine oxides. They have been known in the art for a long time, e.g., C₁₂-C₁₈-alkyl dimethyl amine oxides such as lauryl dimethyl amine oxide, C₁₂-C₁₈-alkyl amidopropyl or -ethyl amine oxides, C₁₂-C₁₈-alkyl di(hydroxyethyl) or (hydroxypropyl) amine oxides, or also amine oxides with ethylene oxide and (or) propylene oxide groups in their alkyl chain.

These amine oxides are sold on the market, e.g., under the trade names "Ammonyx®", "Aromox®", or "Genaminox®".

Especially appropriate nonionic surfactants are C₈-C₁₈-alkyl glucosides with a condensation degree of more than 1 to about 2.5, as disclosed in European Patent No. 70,074.

The compositions according to the invention may also contain amphoteric or zwitterionic surfactants in a proportion from about 0.1% to about 5%, particularly from about 0.5% to about 3% by wt., calculated to the total composition. For this purpose various known betaines such as fatty acid amidoalkyl betaines and sulfobetaines, e.g. lauryl hydroxysulfobetaine, are mentioned; long-chain alkyl amino acids have also proved suitable.

Other additives are complexing agents, dyestuffs, preservatives, pH-regulants, viscosity modifiers, fragrances, pearl gloss agents, thickeners, humectants, plant and animal oils such as jojoba oil, etc.

Particularly suitable additives are hair conditioning actives. As such, especially cationic polymers, preferably in a proportion between 0.05% to 2.5%, particularly 0.2% to 1.5% by wt. of the total composition, are used.

European Patent No. 337,354 discloses the use of cationic polymers together with alkyl polyglucoside surfactants; the cationic polymers listed therein on pp.3 to 7 are also appropriate conditioning additives in the compositions of the invention; equally useful are the products known under the generic name "Polyquaternium" as listed in "CTFA International Cosmetic Ingredient Dictionary".

Further conditioning additives are the well-known protein hydrolyzates, e.g., in a quantity from 0.25 to 5%, preferably 0.5% to 2.5% by wt. of the total composition.

Water-soluble collagen or collagen derivatives may also be used.

Eventually, various polysiloxanes may be added as well as conditioning agents in the compositions according to the invention. Their preferred proportion is from about 0.25% to about 5%, particularly from 0.5% to 2.5% by wt. of the total composition. Suitable are both highly volatile and less volatile cyclic or linear polysiloxanes, e.g., silicone oils known under the generic names "Dimethicone" or "Phenyldimethicone" and "Cyclomethicone".

The emulsions or dispersions according to the invention may optionally also be packaged as (foam) aerosols by the addition of inert gases.

The following examples illustrate the compositions according to the invention; these are supposed to remain on the hair after application or, alternatively, after treatment the hair may be rinsed with water.

| **Hair Rinses** | | | | | |
|---|---|---|---|---|---|
| **Examples No. (figures in % by weight)** | **1** | **2** | **3** | **4** | **5** |
| Stearyl trimethyl ammonium chloride | 0.70 | 1.00 | 0.50 | 0.40 | 1.00 |
| Dimethyl dicetyl ammonium chloride | 0.25 | 0.20 | 0.10 | 0.10 | 0.20 |
| Cetyl stearyl alcohol | 2.50 | 2.50 | 2.00 | 2.00 | 2.50 |
| Silicone oil | 0.30 | 0.30 | 0.40 | 0.40 | 0.30 |
| 1,2-Propanediol | 3.00 | 3.00 | 3.00 | 3.00 | - |
| C₁₂-C₁₄-Alkyl polyglucoside (P.D.: ∼ 1.5) | 2.00 | 2.00 | 2.00 | 2.00 | 2.00 |
| Benzophenone-4 | - | - | 0.35 | - | - |
| Benzophenone-9 | - | 0.30 | - | 0.30 | - |
| Benzophenone-5 | 0.30 | - | - | - | 0.30 |
| Hydroxyethyl cellulose | 0.70 | 0.80 | 0.80 | 0.80 | 0.80 |
| Perfume | 0.30 | 0.50 | 0.20 | 0.30 | 0.40 |
| Wheat germ oil | 0.30 | - | - | - | - |
| Locust blossom honey | - | 0.10 | - | - | - |
| Phytantriol | 0.10 | - | - | - | - |
| Ethoxydiglycol | - | - | - | - | 5.00 |
| Lime extract | - | - | 0.20 | - | - |
| Oat meal extract | - | - | 0.30 | - | - |
| Almond oil | - | - | - | - | 0.10 |
| Peach kernel oil | - | - | - | 0.50 | - |
| Camomile extract | - | - | - | 0.20 | 0.10 |
| Water ad | 100.00 | 100.00 | 100.00 | 100.00 | 100.00 |

## Claims

1. Use of an aqueous emulsion or dispersion, comprising
a) at least one surface-active quaternary ammonium compound;
b) at least one fatty or oily substance; and
c) at least one water-soluble UV absorbent material,
as hair conditioning composition.

2. Use of an aqueous emulsion or dispersion according to claim 1, comprising 0.1% to 10% by wt., calculated to the total composition, of at least one surface-active quaternary ammonium compound.

3. Use of an aqueous emulsion or dispersion according to claim 1 or 2, comprising 0.05% to 5% by wt., calculated to the total composition, of at least one water-soluble UV absorbent material.

4. Use of an aqueous emulsion or dispersion according to one or more of claims 1 to 3, comprising as water-soluble UV absorbent material, at least one compound selected from the group of 5-benzoyl-4-hydroxy-2-methoxybenzene sulfonic acid, 2,2'-dihydroxy-4,4'-dimethoxy-3,3'-disulfobenzophenone and (or) phenyl benzimidazole sulfonic acid or the water-soluble salts thereof.

5. Use of an aqueous emulsion or dispersion according to one or more of claims 1 to 4, comprising 0.5% to 5% by wt., calculated to the total composition, of at least one nonionic surfactant.

6. Use of an aqueous emulsion or dispersion according to claim 5, comprising as nonionic surfactant a C₈-C₁₈-alkyl polyglucoside having a polymerization degree of more than 1 to 2.5.
